# EUROPÄISCHE PATENTSCHRIFT

(11) **EP 1 649 899 B1**
(45) Veröffentlichungstag und Bekanntmachung des Hinweises auf die Patenterteilung: **13.08.2008**
(21) Anmeldenummer: 05108970.4
(22) Anmeldetag: 28.09.2005
(51) Int. Cl.: A61Q 5/10, A61K 8/34, A61K 8/60, A61K 8/97, A61Q 5/06

(54) **Mittel und Zubereitung zum Färben von Haaren**
Hair dyeing compositions
Compositions de teinture des cheveux

(30) Priorität: 22.10.2004 DE 202004016367 U
(43) Veröffentlichungstag der Anmeldung: 26.04.2006
(73) Patentinhaber: TANA-Cosmetics MANOA Kurt Fortmann GmbH & CO. KG, 33602 Bielefeld (DE)
(72) Erfinder: Ebeling, Bernd, 37639, Bevern (DE); Fortmann, Ronald, 33604, Bielefeld (DE)
(74) Vertreter: Dantz, Jan Henning

(56) Entgegenhaltungen:
- AU-B- 6 143 080
- AU-B2- 537 805
- GB-A- 211 178
- US-A- 3 194 734
- US-A- 4 963 591
- PATENT ABSTRACTS OF JAPAN Bd. 2003, Nr. 12, 5. Dezember 2003 (2003-12-05) & JP 2003 231620 A (T HASEGAWA CO LTD), 19. August 2003 (2003-08-19)
- DATABASE WPI Section Ch, Week 198806 Derwent Publications Ltd., London, GB; Class A23, AN 1988-040166 XP002364656 & JP 62 299586 A (TORAY IND INC) 26. Dezember 1987 (1987-12-26)

## Beschreibung

Die vorliegende Erfindung betrifft eine Zubereitung und ein Verfahren zum Färben von Haaren, insbesondere von Wimpern und Augenbrauen.

Aus der DE 298 05 893 ist ein Mittel für die oxidative Färbung von Haaren bekannt, das unmittelbar vor der Anwendung durch Vermischen einer Entwicklerlotion mit einer Farbmasse vermischt wird und diese auf einen sauren pH-Wert eingestellt ist. Durch ein solches oxidatives Färben von Haaren lässt sich zwar Haarfärbemittel bereitstellen, das einen pH-Wert zwischen 5 und 7 besitzt, allerdings ist das Herstellungsverfahren relativ aufwendig und auch die Einwirkzeit ist vergleichsweise lange, sodass das Haarfärbemittel sich nur schlecht zum Färben von Wimpern oder Augenbrauen eignet. Ein solches oxidatives Färben mit Wasserstoffperoxid und Farbstoffen ist nur gewerblich anwendbar.

Ferner ist aus der DE 203 14 464 eine Kosmetikzubereitung bekannt, mit der dekorativ Teile der Haut oder Haare gefärbt werden können. Solche dekorativen Mittel besitzen jedoch nur eine begrenzte Haltbarkeit und müssen daher häufig erneuert werden, was als aufwendig empfunden wird.

Aus der AU-B-61430/80 ist ein Mittel zum Färben von Haaren gezeigt, das ein Färbemittel, Ammoniak, Alkohol, Tanninsäure und ein Shampoo umfasst. Das Mittel zum Färben von Haaren wird dabei auf die Haare aufgetragen, um die gewünschte Einfärbung zu erhalten. Eine Zubereitung zum Färben von Haaren in einem zweistufigen Prozess ist nicht gezeigt.

US 3 194 734 offenbart Haarfatbezusammensetzungen die Ammoniak und Silvermitrat enthalten.

Es ist daher Aufgabe der vorliegenden Erfindung eine Zubereitung und ein Verfahren zum Färben von Haaren, insbesondere von Wimpern und Augenbrauen zu schaffen, die eine lang anhaltende Wirkung besitzen und einfach zu verwenden sind.

Diese Aufgabe wird mit einer Zubereitung mit den Merkmalen des Anspruches 1 und einem Verfahren mit den Merkmalen des Anspruches 10 gelöst.

Erfindungsgemäß umfasst die Zubereitung ein Mittel zum Färben von Haaren mit einer wässrigen Lösung mit Tannin (Gerbsäure) und Ammoniak. Durch dieses Mittel werden die entsprechenden Haare aufgeweicht und können dann auf einfache Weise mit Silbernitratgel oder anderen Färbemitteln gefärbt werden. Durch die zweistufige Ausgestaltung des Färbeprozesses, nämlich der Aufweichung der Haare und der anschließenden Färbung kann die Einwirkzeit erheblich verkürzt werden, sodass in nur wenigen Minuten eine Färbung der Haare erhalten wird. Durch die wässrige Lösung mit Tannin und Ammoniak wird jedoch auch ein Einsatz im Augenbereich ermöglicht. Durch das Tannin werden die Haare teilweise vorgefärbt, wobei insbesondere das Aufweichen der Haare für das anschließende Färben ein Vorteil ist.

Vorzugsweise ist in der wässrigen Lösung auch ein Anteil Natriumsulfit enthalten. Die Anteile der Stoffe Tannin, Ammoniak und Natriumsulfit liegen jeweils unter 10 Gew % und die wässrige Lösung besitzt einen pH-Wert von unter 10, um ein Färben auch im Bereich der Augen zu ermöglichen. Denn Tränenflüssigkeit besitzt einen basischen pH-Wert im Bereich von 7,4 und der Einsatz starker Basen und Säuren verbietet sich im Bereich der Augen.

Vorzugsweise wird das Mittel zum Färben von Haaren zusammen mit einem Silbernitratgel verwendet, das nach dem Aufweichen der Haare durch das Mittel mit Tannin und Ammoniak auf die entsprechenden Haare aufgetragen wird. Je nach Einwirkdauer kann dann an den entsprechenden Haaren ein Braun- oder Schwarzton erzeugt werden.

Die Erfindung wird nachfolgend anhand zweier Ausführungsbeispiele beschrieben.

### Beispiel 1

Ein Mittel zum Färben von Haaren besitzt folgende Zusammensetzung:

| | |
|---|---|
| Wasser | 97,82 Gew % |
| Tannin | 0,75 Gew % |
| Natriumsulfit | 0,11 Gew % |
| NH4OH | 1,22 Gew % |
| Waschaktive Substanzen | 0,1 Gew % |

Das Wasser, Tannin und Natriumsulfit werden unter Umgebungstemperatur solange gerührt, bis die wässrige Lösung klar ist. Anschließend wird der Ammoniak untergerührt.

Das so hergestellte Mittel zum Färben von Haaren wird auf die Haare aufgetragen und kann dort für einen Zeitraum zwischen 30 Sekunden und 5 Minuten vorzugsweise 1 bis 2 Minuten einwirken. Anschließend werden die so vorbehandelten Haare mit einem Silbernitratgel bestrichen und somit braun oder schwarz gefärbt. Die zwei überschüssigen Komponenten werden anschließend von den Haaren abgewaschen.

### Beispiel 2

Ein Mittel zum Färben von Haaren umfasst folgende Inhaltsstoffe:

| | |
|---|---|
| Wasser | 87,50 Gew % |
| Tannin | 6,63 Gew % |
| Natriumsulfit | 0,45 Gew % |
| NH4OH (25%) | 4,42 Gew % |
| Waschaktive Substanzen | 1,00 Gew % |

Der wässrigen Lösung aus Tannin und Natriumsulfit wird Ammoniak beigemengt und diese Mischung wird wie bei dem ersten Beispiel auf die Haare aufgetragen, und dient neben der Färbung der Oberflächenbehandlung. Anschließend kann dann eine Färbung mit Silbernitratgel erfolgen.

Das Mittel zum Färben von Haaren kann auch eine andere Mischung von Tannin, Natriumsulfit und Ammoniak aufweisen. Tannin kann in einem Anteil von 0,75 bis 7 Gew %, Ammoniak in einem Anteil von 1 bis 5 Gew % und Natriumsulfit in einem Anteil von 0,1 bis 0,5 Gew % enthalten sein.

Das Mittel zum Färben von Haaren kann ferner waschaktive Substanzen zur Reinigung (Entfettung) und Benetzung der Haare aufweisen, wobei diese in einem Anteil von unter 1 % vorhanden sind.

Ferner ist es möglich, dem Mittel zum Färben von Haaren auch Zitronensäure beizugeben, sodass eine wässrige Lösung im sauren Bereich hergestellt wird. Die Menge an Zitronensäure kann 1 bis 6 Gew % ausmachen.

Das Mittel zum Färben von Haaren kann zudem einen Inhaltsstoff aufweisen, um das Mittel gelig zu machen, also die Viskosität zu erhöhen. Ein solcher Stoff ein Carbomer sein, beispielsweise das unter dem Handelsnamen vertriebene Carbopol 1382, wobei das Carbomer in einem Anteil zwischen 0,1 bis 1 Gew%, vorzugsweise 0,3 bis 0,5 Gew% zugegeben wird.

Zum Färben der Haare wird anschließend vorzugsweise Silbernitratgel (AgNO₃) aufgetragen.

## Patentansprüche

1. Zubereitung zum Färben von Haaren, insbesondere von Wimpern und Augenbrauen, mit einem Mittel zum Färben und Aufweichen von Haaren, umfassend eine wässrige Lösung mit Tannin und Ammoniak, und einem Mittel zum Färben von Haaren in Form von Silbernitratgel.

2. Zubereitung nach Anspruch 1, **dadurch gekennzeichnet, dass** in der wässrigen Lösung ein Anteil Natriumsulfit enthalten ist.

3. Zubereitung nach Anspruch 1 oder 2, **dadurch gekennzeichnet, dass** Tannin in einem Anteil zwischen 0,75 und 7 Gew %, vorzugsweise zwischen 2 und 5 Gew % in dem Mittel enthalten ist.

4. Zubereitung nach einem der Ansprüche 1 bis 3, **dadurch gekennzeichnet, dass** Ammoniak in einem Anteil zwischen 1 und 5 Gew %, vorzugsweise zwischen 2 und 4 Gew % in dem Mittel enthalten ist.

5. Zubereitung nach einem der Ansprüche 1 bis 4, **dadurch gekennzeichnet, dass** Natriumsulfit in einem Anteil zwischen 0,1 und 0,5 Gew %, vorzugsweise 0,2 bis 0,4 Gew % enthalten ist.

6. Zubereitung nach einem der Ansprüche 1 bis 5, **dadurch gekennzeichnet, dass** der pH-Wert der wässrigen Lösung kleiner als 10, vorzugsweise zwischen 7 und 9 ist.

7. Zubereitung nach einem der Ansprüche 1 bis 6, **dadurch gekennzeichnet, dass** die wässrige Lösung waschaktive Substanzen mit einem Anteil unter 1 Gew % enthält.

8. Zubereitung nach einem der Ansprüche 1 bis 7, **dadurch gekennzeichnet, dass** als weiterer Inhaltsstoff in dem Mittel ein Stoff zur Erhöhung der Viskosität, vorzugsweise ein Carbomer, enthalten ist.

9. Zubereitung nach Anspruch 8, **dadurch gekennzeichnet, dass** ein Carbomer in einem Anteil von 0,1 bis 1 Gew % enthalten ist.

10. Verfahren zum Färben von Haaren, insbesondere Wimpern und Augenbrauen, mit einem Mittel zum Färben und Aufweichen von Haaren, umfassend eine wässrige Lösung mit Tannin und Ammoniak, das zum Aufweichen auf Haare aufgebracht wird, und einem anschließenden Färben der Haare mit einem Silbernitratgel.

## Claims

1. Preparation for dyeing hair, in particular eyelashes and eyebrows, with a composition for dyeing and softening hair comprising an aqueous solution with tannin and ammonia, and a composition for dyeing hair in the form of silver nitrate gel.

2. Preparation according to Claim 1, **characterized in that** an amount of sodium sulphite is present in the aqueous solution.

3. Preparation according to Claim 1 or 2, **characterized in that** tannin is present in the composition in an amount between 0.75 and 7% by weight, preferably between 2 and 5% by weight.

4. Preparation according to one of Claims 1 to 3, **characterized in that** ammonia is present in the composition in an amount between 1 and 5% by weight, preferably between 2 and 4% by weight.

5. Preparation according to one of Claims 1 to 4, **characterized in that** sodium sulphite is present in an amount between 0.1 and 0.5% by weight, preferably 0.2 to 0.4% by weight.

6. Preparation according to one of Claims 1 to 5, **characterized in that** the pH of the aqueous solution is less than 10, preferably between 7 and 9.

7. Preparation according to one of Claims 1 to 6, **characterized in that** the aqueous solution comprises washing-active substances in an amount below 1% by weight.

8. Preparation according to one of Claims 1 to 7, **characterized in that** a substance for increasing the viscosity, preferably a carbomer, is present as further ingredient in the composition.

9. Preparation according to Claim 8, **characterized in that** a carbomer is present in an amount from 0.1 to 1% by weight.

10. Method of dyeing hair, in particular eyelashes and eyebrows, with a composition for dyeing and softening hair, comprising an aqueous solution with tannin and ammonia, which is applied to hair for softening, and subsequent dyeing of the hair with a silver nitrate gel.

## Revendications

1. Préparation pour teindre les cheveux, en particulier les cils et les sourcils, avec un agent de teinture et d'assouplissement des cheveux, comprenant une solution aqueuse comportant un tanin et de l'ammoniaque, et un agent de teinture des cheveux sous la forme de gel de nitrate d'argent.

2. Préparation selon la revendication 1, **caractérisée en ce qu'**une proportion de sulfite de sodium est contenue dans la solution aqueuse.

3. Préparation selon la revendication 1 ou 2, **caractérisée en ce que** le tanin est contenu dans l'agent en une proportion entre 0,75 et 7 % en poids, de préférence entre 2 et 5 % en poids.

4. Préparation selon l'une quelconque des revendications 1 à 3, **caractérisée en ce que** l'ammoniaque est contenue dans l'agent en une proportion entre 1 et 5 % en poids, de préférence entre 2 et 4 % en poids.

5. Préparation selon l'une quelconque des revendications 1 à 4, **caractérisée en ce que** le sulfite de sodium est contenu en une proportion entre 0,1 et 0,5 % en poids, de préférence entre 0,2 et 0,4 % en poids.

6. Préparation selon l'une quelconque des revendications 1 à 5, **caractérisée en ce que** le pH de la solution aqueuse est inférieur à 10, de préférence entre 7 et 9.

7. Préparation selon l'une quelconque des revendications 1 à 6, **caractérisée en ce que** la solution aqueuse contient des substances détergentes en une proportion inférieur à 1 % en poids.

8. Préparation selon l'une quelconque des revendications 1 à 7, **caractérisée en ce que** l'agent contient comme autre ingrédient, une substance pour augmenter la viscosité, de préférence un carbomère.

9. Préparation selon la revendication 8, **caractérisée en ce qu'**un carbomère est contenu en une proportion de 0,1 à 1 % en poids.

10. Procédé de teinture des cheveux, en particulier des cils et des sourcils, avec un agent de teinture et d'assouplissement des cheveux, comprenant une solution aqueuse comportant un tanin et de l'ammoniaque, qui est appliqué sur les cheveux pour les assouplir, et ensuite une teinture des cheveux avec un gel de nitrate d'argent.
